# FASCICULE DE BREVET EUROPEEN

(11) **EP 0 876 331 B1**
(45) Date de publication et mention de la délivrance du brevet: **12.06.2002**
(21) Numéro de dépôt: 96944089.0
(22) Date de dépôt: 27.12.1996
(51) Int. Cl.: C07C 241/02

(54) **PROCEDE POUR LA SYNTHESE D'ARYLHYDRAZINE PAR REDUCTION D'UN DERIVE DIAZOIQUE**
VERFAHREN ZUR HERSTELLUNG VON ARYLHYDRAZIN DURCH REDUKTION VON AZO-DERIVATE
METHOD FOR SYNTHESIZING ARYL HYDRAZINE THROUGH REDUCTION OF A DIAZO COMPOUND DERIVATIVE

(30) Priorité: 29.12.1995 FR 9515711
(43) Date de publication de la demande: 11.11.1998
(73) Titulaire: RHODIA CHIMIE, 92408 Courbevoie Cédex (FR)
(72) Inventeur: MAS, Jean-Manuel, F-69330 Millery (FR); ROCHIN, Christophe, F-69006 Lyon (FR)
(74) Mandataire: Ricalens, François
(86) Numéro de dépôt international: FR9602089
(87) Numéro de publication internationale: WO9724316

(56) Documents cités:
- EP-A- 0 723 953
- DE-A- 3 901 705
- CHEMISCHE BERICHTE, vol. 93, 1960, WEINHEIM DE, pages 540-544, XP002014817 R. HUISGEN ET AL: "Zum Mechanismus der Phenylhydrazinsynthese nach E. Fischer"

## Description

La présente invention a pour objet un procédé pour la synthèse d'arylhydrazine ou de dérivé, par réduction d'un dérivé diazoïque. Elle concerne plus particulièrement une réduction de type bisulfitique.

La voie d'accès la plus directe aux hydrazines substituées par un dérivé arylique est dans la plupart des cas la diazotation d'une aniline, suivie d'une réduction en général hydrogénation.

Cette voie présente, outre un coût relativement élevé, de nombreuses difficultés parmi lesquels on peut citer un nombre d'étapes souvent prohibitif, la formation de sous produits lourds entraînant ipso facto un rendement global et une pureté des plus médiocres. Brochant sur le tout, les techniques de réduction donnent des résultats erratiques, selon les substituants sur les noyaux. Le problème est particulièrement net dans le cas des noyaux aromatiques appauvris en électron.

Ainsi, au cours de l'étude qui a mené à la présente invention, Il a été montré que la technique la plus classique pour réduire les "diazo", à savoir l'utilisation d'étain donnait des résultats dont le moins que l'on puisse dire est qu'ils sont médiocres.

C'est pourquoi un des buts de la présente invention est de fournir un procédé qui permette l'obtention d'arylhydrazine à partir de dérivé diazoïque.

Un autre but de la présente invention est de fournir un procédé qui soit adapté à des noyaux faiblement ou moyennement appauvris en électrons.

Un autre but de la présente invention est de fournir un procédé qui permette de bons rendements.

Un autre but de la présente invention est de fournir un procédé qui permette en une étape l'obtention d'arylhydrazines facilement purifiable, voire directement de grande pureté.

Ces buts et d'autres qui apparaîtront par la suite sont atteints au moyen d'un **procédé pour la synthèse d'arylhydrazine par réduction d'un sel de diazonium, caractérisé par le fait qu'il** comporte au moins l'étape suivante :

c) mise en contact du dérivé diazoïque avec une solution de sulfite dont le pH est au moins égal à 7 **et est tamponnée par l'ammoniaque**.

En effet l'utilisation de sulfite s'est révélée particulièrement intéressante comme réducteur à condition que l'on se place à un pH suffisamment basique. Ceci est particulièrement surprenant car la réaction de réduction nécessite la présence d'ion acide.

**Le tamponnage au moyen d'ammoniaque est particulièrement surprenant si l'on se réfère au document Chemische Berichte, vol. 93, 1960, Weinheim DE, pages 540-544, où il est clairement indiqué la présence de sodium (sous forme de chlorure) permettrait une amélioration très significative de la cinétique**.

Avantageusement, pour éviter la présence de produit coloré altérant la pureté et la couleur de l'hydrazine, et donc pour éviter une étape de purification poussée, il est souhaitable de se placer à un pH compris entre 7,2 et 11, de préférence entre 7,5 et 10.

Pour éviter la présence de goudron et de lourds, il est préférable que la température à la quelle est menée l'étape c) soit au plus égale à 100°C, avantageusement à 50°C.

Sans que l'on puisse complètement l'expliquer, les meilleurs résultats sont obtenus lorsque le pH de la solution de sulfite/bisulfite est tamponné par l'ammoniaque.

Selon une mise en oeuvre particulièrement avantageuse de la présente invention, l'étape c) est réalisée par l'addition dudit dérivé diazoïque dans un pied de solution réductrice bisulfitique.

Il est souhaitable que pendant la durée de l'étape c), le pH soit régulé de manière qu'il soit toujours proche d'une valeur choisie à l'avance et supérieure à 7 et inférieure à 11, avantageusement inférieure à 10.

Le procédé comporte avantageusement en outre une étape a) de synthèse du dérivé diazoïque, en particulier pour éviter l'entreposage du dérivé diazoïque à réduire.

Cette étape a) de synthèse du dérivé diazoïque est, en général, réalisée par l'action d'un nitrite, avantageusement métallique ou d'alcoyle, sur une aniline.

Avantageusement ledit nitrite est un nitrite alcalin.

En général, ledit dérivé diazoïque est un sel de diazonium.

Les résultats sont plus prédictibles, si le noyau aromatique porteur de la fonction diazoïque est un cycle benzénique.

Le procédé est particulièrement intéressant lorsque ledit cycle benzénique est compte non tenu de la fonction diazoïque présente des substituants tels qu'il est globalement appauvri en électron ou, de préférence et, lorsque le cycle benzénique est, compte non tenu de la fonction diazoïque, appauvri en électron par au moins une fonction électroattractrice par effet inducteur. De préférence lorsque les deux conditions sont réunies.

Il est souhaitable que ledit cycle benzénique ne présente pas de substituant électroattracteur par effet mésomère.

Lorsque le noyau porte des substituants on peut évaluer la richesse en électron du noyau à l'aide des constantes σₚ dites de "Hammett". Ainsi la somme des constantes de Hammett des substituants dudit cycle benzénique, compte non tenu de la fonction diazoïque, est comprise entre 0,1 et 0,7 ; avantageusement entre 0,1 et 0,5 ; de préférence entre 0,1 et 0,35.

Pour plus de détails sur les constantes de Hammett on peut se référer à la troisième édition du manuel écrit par monsieur le professeur Jerry March "advanced organic chemistry" (pages 242 à 250) et édité par John Wiley and sons.

Lorsque le cycle benzénique ne présente qu'un substituant électroattracteur, ledit substituant électroattracteur l'est principalement par effet inducteur. En outre il est souhaitable que ledit substituant électroattracteur soit situé en position ortho ou para de la fonction diazoïque.

Ces substituants électroattracteurs sont avantageusement choisis parmi les halogènes légers (chlore et/ou de préférence fluor), le nitrile et les perhalogénoalcoyles.

Pour éviter la dégradation du dérivé diazoïque dans le mélange réactionnel et donc pour éviter d'avoir à séparer le "diazo" du mélange réactionnel dont il est issu, II est souhaitable que le procédé comporte en outre un étape b) de réduction de la teneur en nitrite.

Il est également possible de passer directement de l'étape a) à l'étape c) à condition d'éviter l'entreposage et d'utiliser le mélange réactionnel rapidement après l'étape a). Cela est le cas dans les procédés continu ou semi continu.

Avantageusement ladite étape b) de réduction de la teneur en nitrite est réalisée en mettant en contact le mélange réactionnel issu de l'étape a) avec un réducteur de la fonction nitrite, avantageusement un acide-amide, de préférence l'acide sulfamique et/ou un de ses dérivés.

Pour parfaire le processus, et si le dérivé hydrazinique obtenu dans l'étape c) n'est pas utilisable tel quel, le procédé comporte en outre un étape d) d'hydrolyse du dérivé sulfoné obtenu à l'étape c).

Cette étape d) d'hydrolyse du dérivé sulfoné est avantageusement réalisée en milieu acide, de préférence en milieu acide chlorhydrique. Laquelle étape d) d'hydrolyse du dérivé sulfoné est réalisée à température comprise entre 20°C et 70° C, de préférence entre 40 et 60°C.

Les exemples non limitatifs suivants illustrent l'invention.

### PREPARATION DE 2-FLUOROPHENYLHYDRAZINE

### SALIFICATION

Dans un réacteur de 6 litres à double enveloppe, inerté à l'azote et équipé d'une agitation mécanique, d'un réfrigérant, d'une ampoule de coulée et d'une sonde thermométrique on charge 400 g d'eau distillée puis on coule, en environ 30 minutes, 470 ml d'une solution aqueuse à 32.5 % d'acide chlorhydrique.

La température de la solution est portée à 80°C puis on coule en une heure 224 g (2 moles) de Fluoro-2 aniline (à 99 %). Maintien d'une 1/2 heure dans ces conditions.

### DIAZOTATION

La solution de chlorhydrate de Fluoro-2 aniline est refroidie à 0°C. Le chlorhydrate précipite et on obtient une bouillie blanche agitable.

On additionne alors, en 2h30 et de manière à ne pas dépasser 3°C dans la masse, 363 g d'une solution aqueuse à 40 % de nitrite de sodium. Le milieu devient homogène et prend une coloration rouge-orangée.

Après une finition de 30 minutes on coule très lentement 97 g d'une solution aqueuse à 12.5 % d'acide sulfamique pour détruire l'excès de nitrite de sodium. Le dégagement d'azote provoque la formation de mousses. Finition d'une 1/2 heure puis soutirage du milieu.

### REDUCTION

Après avoir préparé la solution réductrice par addition en une 1/2 heure de 285 g d'une solution d'ammoniaque à 28 % sur 1547 g d'une solution aqueuse à 40 % de bisulfite de sodium et ramené le milieu à une température de 25°C, on coule la solution de diazo en 2 heures. Le milieu réactionnel est ensuite chauffé à 50°C pendant 30 minutes.

### HYDROLYSE

Après ce maintien on coule, en environ 3 heures, 1123 g d'une solution aqueuse à 30 % d'acide chlorhydrique. Le milieu homogène et jaune-orangé est maintenu 30 minutes à 50°C.

Nota : il est possible à ce niveau de réaliser une extraction au toluène pour éliminer certains produits lourds qui auraient pu être formés.

### NEUTRALISATION

La libération de l'hydrazine base est alors réalisée par coulée lente de 1250 ml d'une solution aqueuse à 30.5 % de soude. Le milieu est ensuite extrait par 450 ml de toluène suivis par 2 x 300 ml de toluène. Les phases organiques sont rassemblées.

### DESOLVATATION

La désolvatation est réalisée par stripping à l'azote (70°C sous 10 mm Hg) après distillation partielle du toluène, soit par distillation sur appareil de type "Luwa" en opérant à 70°C sous 5 mm Hg.

On obtient ainsi 241 g de produit brut titrant 96.1 % par analyse CLHP.

Rendement en produit isolé = 92 %

## Revendications

1. Procédé pour la synthèse d'arylhydrazine par réduction d'un sel de diazonium , **caractérisé par le fait que** qu'il comporte au moins l'étape suivante :
c) mise en contact du dérivé diazoïque avec une solution de sulfite dont le pH est au moins égal à 7 et est tamponnée par l'ammoniaque.

2. Procédé selon la revendication 1, **caractérisé par le fait que** ledit pH est compris entre 7,2 et 11.

3. Procédé selon les revendications 1 et 2, **caractérisé par le fait que** ledit pH est compris entre 7,5 et 10.

4. Procédé selon les revendications 1 à 3, **caractérisé par le fait que** la température à la quelle est menée l'étape c) est au plus égale à 100°C.

5. Procédé selon la revendication 4, **caractérisé par le fait que** ladite température est au plus égale à 50°C.

6. Procédé selon les revendications 1 à 5, **caractérisé par le fait que** l'étape c) est réalisée par l'addition dudit dérivé diazoïque dans un pied de solution réductrice bisulfitique.

7. Procédé selon les revendications 1 à 6, **caractérisé par le fait que** pendant la durée de l'étape c), le pH est régulé de manière qu'il soit toujours proche d'une valeur supérieure à 7 et inférieure à 11, avantageusement à 10.

8. Procédé selon les revendications 1 à 7, **caractérisé par le fait que** qu'il comporte en outre une étape a) de synthèse du sel de diazonium.

9. Procédé selon la revendication 8, **caractérisé par le fait que** ladite étape a) de synthèse du dérivé diazoïque est réalisée par l'action d'un nitrite, avantageusement métallique ou d'alcoyle, sur une aniline.

10. Procédé selon la revendication 9, **caractérisé par le fait que** ledit nitrite est un nitrite alcalin.

11. Procédé selon les revendications 1 à 10, **caractérisé par le fait que** ledit dérivé diazoïque est un sel de diazonium.

12. Procédé selon les revendications 1 à 11, **caractérisé par le fait que** le noyau aromatique porteur de la fonction diazoïque est un cycle benzénique.

13. Procédé selon la revendication 12, **caractérisé par le fait que** ledit cycle benzénique est, compte non tenu de la fonction diazoïque, appauvri en électron par au moins une fonction électroattractrice par effet inducteur.

14. Procédé selon les revendications 12 et 13, **caractérisé par le fait que** ledit cycle benzénique est compte non tenu de la fonction diazoïque présente des substituants tel qu'il est globalement appauvri en électron.

15. Procédé selon les revendications 12 à 14, **caractérisé par le fait que** ledit cycle benzénique ne présente pas de substituant électroattracteur par effet mésomère.

16. Procédé selon les revendications 12 à 15, **caractérisé par le fait que** la somme des constantes de Hammett des substituants dudit cycle benzénique est comprise entre 0,1 et 0,7.

17. Procédé selon les revendications 12 à 16, **caractérisé par le fait que** ledit cycle benzénique ne présente qu'un substituant électroattracteur, ledit substituant électroattracteur étant électroattracteur par effet inducteur.

18. Procédé selon les revendications 12 à 17, **caractérisé par le fait que** ledit substituant électroattracteur est situé en position ortho ou para de la fonction diazoïque.

19. Procédé selon les revendications 1 à 18, **caractérisé par le fait que** les substituants électroattracteurs sont choisis parmi les halogènes légers (chlore et/ou de préférence fluor), le nitrile et les perhalogénoalcoyles.

20. Procédé selon les revendications 1 à 19, **caractérisé par le fait que** le procédé comporte en outre un étape b) de réduction de la teneur en nitrite.

21. Procédé selon la revendication 20, **caractérisé par le fait que** ladite étape b) de réduction de la teneur en nitrite est réalisée en mettant en contact le mélange réactionnel issu de l'étape a) avec un réducteur de la fonction nitrite, avantageusement un acide-amide, de préférence l'acide sulfamique et ou un de ses dérivés.

22. Procédé selon les revendications 1 à 21, **caractérisé par le fait que** le procédé comporte en outre un étape d) d'hydrolyse du dérivé sulfoné obtenu à l'étape c).

23. Procédé selon la revendication 22, **caractérisé par le fait que** l'étape d) d'hydrolyse du dérivé sulfoné est réalisée en milieu acide, de préférence en milieu acide chlorhydrique..

24. Procédé selon les revendications 22 et 23, **caractérisé par le fait que** l'étape d) d'hydrolyse du dérivé sulfoné est réalisée à température comprise entre 20°C et 70° C, de préférence entre 40 et 60°C.

## Claims

1. Process for the synthesis of arylhydrazine by reduction of a diazonium salt, **characterized in that** it includes at least the following step:
c) placing the diazo derivative in contact with a sulphite solution whose pH is at least equal to 7 and which is buffered with aqueous ammonia.

2. Process according to Claim 1, **characterized in that** the said pH is between 7.2 and 11.

3. Process according to Claims 1 and 2, **characterized in that** the said pH is between 7.5 and 10.

4. Process according to Claims 1 to 3, **characterized in that** the temperature at which step c) is carried out is at most equal to 100°C.

5. Process according to Claim 4, **characterized in that** the said temperature is at most equal to 50°C.

6. Process according to Claims 1 to 5, **characterized in that** step c) is carried out by addition of the said diazo derivative to an initial charge of reductive bisulphite solution.

7. Process according to Claims 1 to 6, **characterized in that** during step c), the pH is adjusted such that it is always close to a value above 7 and below 11, advantageously below 10.

8. Process according to Claims 1 to 7, **characterized in that** it also includes a step a) of synthesis of the diazonium salt.

9. Process according to Claim 8, **characterized in that** the said step a) of synthesis of the diazo derivative is carried out by the action of a nitrite, advantageously an alkyl or metal nitrite, on an aniline.

10. Process according to Claim 9, **characterized in that** the said nitrite is an alkaline nitrite.

11. Process according to Claims 1 to 10, **characterized in that** the said diazo derivative is a diazonium salt.

12. Process according to Claims 1 to 11, **characterized in that** the aromatic ring bearing the diazo function is a benzene ring.

13. Process according to Claim 12, **characterized in that** the said benzene ring is, not taking the diazo function into account, electron-poor owing to an inductive effect by at least one electron-withdrawing function.

14. Process according to Claims 12 and 13, **characterized in that** the said benzene ring, not taking the diazo function into account, has substituents such that it is electron-poor overall.

15. Process according to Claims 12 to 14, **characterized in that** the said benzene ring has no mesomeric-effect electron-withdrawing substituents.

16. Process according to Claims 12 to 15, **characterized in that** the sum of the Hammett constants of the substituents on the said benzene ring is between 0.1 and 0.7.

17. Process according to Claims 12 to 16, **characterized in that** the said benzene ring has only one electron-withdrawing substituent, the said electron-withdrawing substituent being electron-withdrawing by means of an inductive effect.

18. Process according to Claims 12 to 17, **characterized in that** the said electron-withdrawing substituent is located in a position ortho or para to the diazo function.

19. Process according to Claims 1 to 18, **characterized in that** the electron-withdrawing substituents are chosen from light halogens (chlorine and/or preferably fluorine), nitrile and perhaloalkyls.

20. Process according to Claims 1 to 19, **characterized in that** the process also includes a step b) of reduction of the nitrite content.

21. Process according to Claim 20, **characterized in that** the said step b) of reduction of the nitrite content is carried out by placing the reaction mixture obtained in step a) in contact with a reducing agent for the nitrite function, advantageously an amide acid, preferably sulphamic acid and/or a derivative thereof.

22. Process according to Claims 1 to 21, **characterized in that** the process also includes a step d) of hydrolysis of the sulphone derivative obtained in step c).

23. Process according to Claim 22, **characterized in that** the step d) of hydrolysis of the sulphone derivative is carried out in acidic medium, preferably in hydrochloric acid medium.

24. Process according to Claims 22 and 23, **characterized in that** the step d) of hydrolysis of the sulphone derivative is carried out at a temperature of between 20°C and 70°C, preferably between 40 and 60°C.

## Patentansprüche

1. Verfahren zur Synthese von Arylhydrazin durch Reduktion eines Diazoniumsalzes, **dadurch gekennzeichnet, daß** das Verfahren mindestens den folgenden Verfahrensschritt umfaßt:
c) Inkontaktbringen des Diazoderivates mit einer Sulfitlösung, deren pH-Wert mindestens 7 beträgt und die durch eine Ammoniaklösung gepuffert ist.

2. Verfahren nach Anspruch 1, **dadurch gekennzeichnet, daß** der pH-Wert 7,2 bis 11 beträgt.

3. Verfahren nach Anspruch 1 und 2, **dadurch gekennzeichnet, daß** der pH-Wert 7,5 bis 10 beträgt.

4. Verfahren nach den Ansprüchen 1 bis 3, **dadurch gekennzeichnet, daß** die Temperatur, bei der der Verfahrensschritt c) durchgeführt wird, höchstens 100 °C beträgt.

5. Verfahren nach Anspruch 4, **dadurch gekennzeichnet, daß** die Temperatur höchstens 50 °C beträgt.

6. Verfahren nach den Ansprüchen 1 bis 5, **dadurch gekennzeichnet, daß** der Verfahrensschritt c) durchgeführt wird durch Zugabe des Diazoderivates zum Boden der reduzierenden Bisulfitlösung.

7. Verfahren nach den Ansprüchen 1 bis 6, **dadurch gekennzeichnet, daß** während der Dauer des Verfahrensschrittes c) der pH-Wert so eingestellt wird, daß er stets etwa bei einem Wert oberhalb von 7 und unterhalb von 11, vorzugsweise bei 10, liegt.

8. Verfahren nach den Ansprüchen 1 bis 7, **dadurch gekennzeichnet, daß** das Verfahren außerdem einen Verfahrensschritt a) der Synthese des Diazoniumsalzes umfaßt.

9. Verfahren nach Anspruch 8, **dadurch gekennzeichnet, daß** der Verfahrensschritt a) der Synthese der Diazoverbindung durchgeführt wird durch Einwirkung eines Nitrits, vorzugsweise Metall- oder Alkylnitrits, auf ein Anilin.

10. Verfahren nach Anspruch 9, **dadurch gekennzeichnet, daß** das Nitrit ein alkalisches Nitrit ist.

11. Verfahren nach den Ansprüchen 1 bis 10, **dadurch gekennzeichnet, daß** das Diazoderivat ein Diazoniumsalz ist.

12. Verfahren nach den Ansprüchen 1 bis 11, **dadurch gekennzeichnet, daß** der aromatische Kern, der die Diazofunktion trägt, ein Benzolring ist.

13. Verfahren nach Anspruch 12, **dadurch gekennzeichnet, daß** der Benzolring, abgesehen von der Diazofunktion, aufgrund mindestens einer elektronenziehenden Funktion, die über einen induktiven Effekt elektronenziehend wirkt, elektronenarm ist.

14. Verfahren nach den Ansprüchen 12 und 13, **dadurch gekennzeichnet, daß** der Benzolring, abgesehen von der Diazofunktion, solche Substituenten aufweist, daß er insgesamt elektronenarm ist.

15. Verfahren nach den Ansprüchen 12 bis 14, **dadurch gekennzeichnet, daß** der Benzolring keine über Mesomerieeffekte elektronenziehend wirkende Substituenten aufweist.

16. Verfahren nach den Ansprüchen 12 bis 15, **dadurch gekennzeichnet, daß** die Summe der Hammett-Konstanten der Substituenten des Benzolrings zwischen 0,1 und 0,7 liegt.

17. Verfahren nach den Ansprüchen 12 bis 16, **dadurch gekennzeichnet, daß** der Benzolring nur einen elektronenziehenden Substituenten aufweist, wobei der elektronenziehende Substituent aufgrund eines induktiven Effektes elektronenziehend wirkt.

18. Verfahren nach den Ansprüchen 12 bis 17, **dadurch gekennzeichnet, daß** der elektronenziehende Substituent sich in ortho- oder para-Funktion zur Diazofunktion befindet.

19. Verfahren nach den Ansprüchen 1 bis 18, **dadurch gekennzeichnet, daß** die elektronenziehenden Substituenten ausgewählt sind aus leichten Halogenen (Chlor und/oder vorzugsweise Fluor), Nitril und Perhalogenalkylen.

20. Verfahren nach den Ansprüchen 1 bis 19, **dadurch gekennzeichnet, daß** das Verfahren außerdem einen Verfahrensschritt b) der Reduktion des Nitritgehaltes umfaßt.

21. Verfahren nach Anspruch 20, **dadurch gekennzeichnet, daß** der Verfahrensschritt b) der Reduktion des Nitritgehaltes durchgeführt wird, indem man die aus dem Verfahrensschritt a) stammende Reaktionsmischung mit einem Reduktionsmittel in bezug auf die Nitritfunktion, insbesondere einem Säureamid, vorzugsweise Sulfaminsäure oder ihren Derivaten, in Kontakt bringt.

22. Verfahren nach den Ansprüchen 1 bis 21, **dadurch gekennzeichnet, daß** das Verfahren außerdem einen Verfahrensschritt d) der Hydrolyse des sulfonierten Derivates, das im Verfahrensschritt c) erhalten wird, umfaßt.

23. Verfahren nach Anspruch 22, **dadurch gekennzeichnet, daß** der Verfahrensschritt d) der Hydrolyse des sulfonierten Derivates in saurem Milieu, vorzugsweise in salzsaurem Milieu, durchgeführt wird.

24. Verfahren nach den Ansprüchen 22 und 23, **dadurch gekennzeichnet, daß** der Verfahrensschritt d) der Hydrolyse des sulfonierten Derivates bei einer Temperatur zwischen 20 °C und 70 °C, vorzugsweise zwischen 40 °C und 60 °C, durchgeführt wird.
